# NOUVEAU FASCICULE DE BREVET EUROPEEN

(11) **EP 1 040 821 B2**
(45) Date de publication et mention de la décision concernant l'opposition: **12.10.2005**
(45) Mention de la délivrance du brevet: 21.11.2001
(21) Numéro de dépôt: 00400489.1
(22) Date de dépôt: 23.02.2000
(51) Int. Cl.: A61K 7/48

(54) **Composition cosmétique comprenant au moins une phase pulvérulente, un hydratant et un dérivé de galactomannane et utilisations**
Kosmetische Zusammensetzung, die zumindest eine pulverige Phase, ein Feuchthaltemittel und ein Galactomannan-Derivat enthält und ihre Verwendungen
Cosmetic composition comprising at least one powdery phase, a hydrating agent and a galactomannane derivative and its uses

(30) Priorité: 31.03.1999 FR 9904045
(43) Date de publication de la demande: 04.10.2000
(73) Titulaire: L'OREAL, 75008 Paris (FR)
(72) Inventeur: Terren, Nadia, 92340 Bourg-La-Reine (FR); Favre, Sophie, 94550 Chevilly-Larue (FR)
(74) Mandataire: Kromer, Christophe

(56) Documents cités:
- EP-A- 0 803 245
- EP-A- 0 898 960
- EP-A1- 0 781 545
- EP-A2- 0 761 200
- WO-A-97/49376
- WO-A1-00/15180
- WO-A1-97/00665
- WO-A1-99/13820
- DE-C1- 19 721 785
- GB-A- 2 325 963
- US-A- 4 472 297
- Produktspezifikation Sident°12DF (SPLS) der Firma Degussa, 14.05.1993
- Auszug aus dem CTFA-online-Katalog "C1-5 Alkyl Galactomannan"
- Produktinformation "Dow Corning° 3225 C Formulation Aid", Dow Corning, 15.06.1998
- Auszug aus dem Produktkatalog zu Timiron° - Goldfarbene Perlglanzpigmente, Merck KGaA, 17.10.1997
- R.L. Whistler, J.N. BeMiller: Industrial Gums - Polysaccharides and their Derivates, 3. Auflage, 1993, Seiten 181-199, 206-207, 359-361

## Description

La présente invention a pour objet une composition cosmétique de maquillage comprenant un hydratant et un dérivé de galactomannane. Ces compositions sont plus particulièrement des fonds de teint, des anti-cernes, des rouges à lèvres, des produits de maquillage pour le corps, des mascara, des eye-liners.

Les compositions cosmétiques, notamment de maquillage, comprennent parfois des corps gras tels que des huiles et des cires, et une phase particulaire généralement composée de charges et de pigments. Elles peuvent ainsi se présenter, par exemple dans les cas des rouges à lèvres, sous forme de stick ou bâton ou sous forme de pâte souple. Elles sont alors souvent sous la forme d'une composition anhydre. Les compositions de maquillage peuvent également comprendre de l'eau ou une phase hydrophile, et se présenter alors notamment sous forme émulsion huile-dans-eau, eau-dans-huile, émulsion multiple, solution ou gel aqueux, notamment lorsqu'il s'agit d'un fond de teint, de crème teintée.

Ces compositions, qui sont avant tout des produits de maquillage, ont pour vocation première d'embellir la personne humaine en soulignant par exemple les qualités esthétiques et/ou encore en dissimulant les imperfections du visage.

Selon le maquillage que l'on désire obtenir, léger, discret ou au contraire accentué, on apporte au visage tantôt une nuance proche de la carnation naturelle de la personne, tantôt une couleur plus soutenue qui rehausse le teint naturel : on utilise pour cela les fonds de teint. On peut également accentuer certains reliefs en apportant des tons plus soutenus en utilisant les blushes par exemple. Pour rehausser le regard, on utilise des teintes plus tranchantes couvrant toutes les nuances des couleurs. Enfin, on met les lèvres en valeur en leur apportant des couleurs généralement foncées.

Dans tous les cas, le résultat est obtenu grâce à la présence d'une phase pulvérulente comprenant des particules solides au sein de la composition finale, ces particules solides pouvant être par exemple des pigments, des nacres ou encore des charges. Ainsi, la nature de ces particules solides, leur couleur mais surtout leur proportion au sein de chacune de ces compositions sont primordiales pour obtenir la nuance souhaitée et ainsi l'effet désiré.

Un des inconvénients de ces particules solides est qu'elles ont tendance à dessécher la peau. Ainsi, après application du produit de maquillage, l'utilisatrice peut ressentir, par exemple au bout de quelques heures, une sensation de tiraiilement de la peau ou des muqueuses extrêmement désagréable.

Or les femmes d'aujourd'hui désirent des produits de maquillage qui leur donnent bonne mine et qui en même temps hydratent la peau et/ou les muqueuses.

Il serait donc intéressant de disposer d'un produit de maquillage qui non seulement conférerait à la peau et/ou les muqueuses la coloration désirée mais qui également ne déssècherait ni la peau ni les muqueuses, voire les hydraterait.

Le document EP-A-0 898 960 décrit une composition épaissie contenant de la silice pyrogénée et un alkyléther de polysaccharide formé de motifs comportant au moins deux cycles osidiques différents, chaque motif comportant an moins un groupe hydroxyle substitué par une chaîne alkyle hydrocarbonée saturée.

Il a déjà été proposé dans le document EP-A-660696 par exemple d'introduire un agent hydratant dans des compositions de maquillage. Toutefois, la présence d'un tel agent hydratant ne suffit pas toujours à compenser l'effet desséchant dû à la présence de particules solides telles que les charges et/ou les pigments.

II subsiste donc le besoin d'une composition cosmétique de maquillage qui non seulement maquille la peau et /ou les muqueuses mais qui en même temps ne dessèche pas lesdites peau et/ou muqueuses et mieux les hydrate.

Or, la Demanderesse a découvert de façon surprenante qu'en introduisant un dérivé de galactomannane, qui seul ne présente pas de propriétés hydratantes, dans une composition comprenant au moins une phase pulvérulente et au moins une phase aqueuse comprenant au moins un agent hydratant, il était possible de réaliser des compositions de maquillage qui ne dessèchent ni la peau ni les muqueuses et qui ont un pouvoir hydratant amélioré par rapport à la même composition ne comprenant pas ce dérivé de galactomannane.

L'invention a donc pour objet une composition cosmétique de maquillage selon la revendication 1.

Les compositions selon l'invention confèrent au visage un teint particulièrement uniforme, aux lèvres la couleur souhaitée : en même temps, non seulement elles ne dessèchent ni la peau ni les muqueuses mais elles les hydratent.

Ces compositions procurent par ailleurs une agréable sensation de fraîcheur à l'application. Elles présentent de plus une excellente tenue : elles ne migrent pas dans les plis tels que les paupières ou les rides, on n'observe pas d'accumulation de produit dans certaines parties du visage. Elles confèrent à la peau un maquillage homogène, une bonne matité.

L'invention a encore pour objet l'utilisation d'un dérivé de galactomannane dans une composition cosmétique, de maquillage comprenant au moins une phase pulvérulente comprenant des particules solides choisies parmi les pigments, les nacres et/ou leurs mélanges, et au moins une phase aqueuse comprenant un agent hydratant présent en une teneur allant de 0,1 à 20% en poids, par rapport au poids total de la composition, afin de diminuer et/ou limiter l'effet desséchant et/ou améliorer le pouvoir hydratant de ladite composition.

Les compositions selon l'invention trouvent notamment une application particulièrement intéressante dans le domaine du maquillage de la peau y compris du cuir chevelu, des muqueuses, des semi-muqueuses, et des phanères. On entend notamment par muqueuse, la partie interne de la paupière inférieure; par semi-muqueuses, on entend plus particulièrement les lèvres du visage; par phanères, on entend les cils, sourcils, cheveux et ongles. Ainsi, l'invention trouve une application toute particulière dans le domaine des produits de maquillage du visage et de la peau, tels que les fonds de teint.

Un autre objet de l'invention est un procédé de traitement non thérapeutique de maquillage de la peau et/ou des muqueuses, consistant à appliquer sur la peau et/ou les muqueuses une composition telle que définie ci-dessus.

Les compositions selon l'invention comprennent au moins une phase pulvérulente constituée de particules qui sont à l'état solide au sein de la composition finale et qui sont choisies parmi les pigments, les nacres et/ou leurs mélanges. Ces particules solides ne sont pas solubilisées dans la composition finale. Elles ont de préférence une taille moyenne pouvant aller jusqu'à 0,5 mm, et de préférence allant de 5 nanomètres à 200 microns.

Par pigments, il faut comprendre des particules blanches ou colorées, minérales ou organiques, insolubles dans le milieu, destinées à colorer et/ou opacifier la composition.

Les pigments peuvent être présents, éventuellement sous forme de mélange, à raison de 0-30 % en poids, par rapport au poids total de la composition, et de préférence à raison de 2-15 %. Ils peuvent être blancs ou colorés, minéraux et/ou organiques. Ils peuvent être de taille moyenne usuelle, c'est-à-dire allant généralement jusqu'à 500 nanomètres, ou nanométrique, c'est-à-dire de taille moyenne des particules comprise entre 5 et 100 nanomètres.

On peut citer, parmi les pigments et les nanopigments minéraux, les dioxydes de titane, de forme sphérique ou encore en paillettes, les dioxydes de zirconium ou de cérium, ainsi que les oxydes de zinc, de fer ou de chrome, les nanotitanes, le bleu ferrique.

Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.

Ces pigments peuvent être traités de manière à rendre leur surface hydrophobe; ce traitement peut être effectué selon les méthodes connues de l'homme du métier; les pigments peuvent notamment être enrobés par des composés siliconés tels que des polydiméthylsiloxane (PDMS) et/ou par des polymères.

On peut également citer les pigments interférentiels qui sont des agents goniochromatiques susceptibles de produire différentes couleurs selon l'incidence de la lumière et l'angle d'observation, qui peuvent être traités ou enrobés, comme ceux décrits par exemple dans EP-A-815826.

Ces pigments peuvent être présents au sein de la composition selon l'invention sous forme de mélanges.

La phase pulvérulente peut comprendre en plus des charges. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Ces charges sont choisies de façon à conférer à la composition un bon glissant et à permettre un bon étalement de ladite composition.

De préférence, les charges utilisées dans la présente invention ne possèdent pas de propriétés épaississantes. On évitera de préférence les charges apportant de la rugosité, comme par exemple certaines silices pyrogénées. De préférence, les compositions selon l'invention sont exemptes de silice pyrogénée.

Ces charges, qui peuvent être présentes dans la composition à raison de 0-20 % en poids, par rapport au poids total de la composition, de préférence 2-10 %, sont par exemple choisies parmi le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques telles que l'Expancel (Nobel Industrie), les microéponges comme le polytrap (Dow Coming) et les microbilles de résine de silicone (Tospearls de la Société TOSHIBA, par exemple), les oxydes de zinc et de titane, les oxydes de zirconium ou de cérium, le carbonate de calcium précipité, le carbonate et l'hydrocarbonate de magnésium, l'hydroxyapatite, les microsphères de silice creuses (Silica Beads de la Société MAPRECOS), les microcapsules de verre ou de céramique ; les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent également se présenter sous la forme de composés à structure composite, comme par exemple des charges composées de plusieurs couches de matériaux pulvérulents différents : on peut citer les composés SiO₂/TiO₂/ SiO₂, TiO₂/CeO₂/SiO₂, ou encore TiO₂/ZnO/Talc.

Elles peuvent également se présenter sous la forme de paillettes comme les polymères de polyéthylène terephtalate/polyméthacrylate vendus sous la dénomination commerciale "CRYSTALINA 323 0.008 HEX" par la société MEADOWBROOK.

Ces charges peuvent être présentes au sein de la composition selon l'invention sous forme de mélanges.

Par nacres, il faut comprendre des particules irisées qui réfléchissent la lumière.

Les nacres peuvent être présentes dans la composition à raison de 0-20 % en poids, de préférence à un taux élevé de l'ordre de 2-15 % en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré et/ou leurs mélanges.

De préférence, la phase pulvérulente est présente dans la composition à une teneur allant de 0,1 à 50%, de préférence encore de 5 à 30% en poids, par rapport au poids total de la composition.

De préférence, les compositions selon l'invention ne sont pas transparentes, c'est-à-dire qu'on ne peut pas voir les caractères d'une page de journal à travers la composition.

La composition selon invention comprend au moins une phase aqueuse qui comprend au moins un agent hydratant. Cet agent hydratant est de préférence choisi parmi la glycérine, le propylène glycol, l'hexylene glycol, le butylène glycol, le pentylène glycol, le dipropylène glycol, le sorbitol, le mannitol, le xylitol, les polyols, les polyéthylène glycols et/ou leurs mélanges. De préférence encore, cet agent hydratant est la glycérine.

L'agent hydratant est présent dans les compositions de l'invention à une teneur allant de 0,1 à 20 %, en poids, par rapport au poids total de la composition, de préférence de 2 à 10% en poids, par rapport au poids total de la composition.

La phase aqueuse des compositions de l'invention comprend également un dérivé de galactomannane. Par dérivé de galactomannane, on entend, au sens de la présente invention, un polysaccharide d'origine naturelle et végétale, hydrophile, comprenant essentiellement une répétition de motifs β-D-mannoses formant une chaîne principale sur laquelle sont branchés des motifs α-D-galactoses. Parmi ces dérivés de galactomannane, on peut citer les dérivés du guar provenant de la gomme de guar et qui comprennent un α-D-galactose pour deux β-D-mannoses et les dérivés de la gomme de caroube qui comprennent un α-D-galactose pour quatre β-D-mannoses.

De préférence, le dérivé de galactomannane utilisé dans la présente invention est un dérivé de guar. Il peut ainsi être choisi parmi les dérivés cationiques de la guar comme les produits vendus sous les dénominations commerciales "Jaguar C13S" et "Jaguar C162" par la Société Rhône-Poulenc, ou encore parmi les dérivés non ioniques de la guar comme l'hydroxy propyl guar vendue sous la dénomination commerciale "Jaguar HP-105" par la Société Rhône-Poulenc, ou encore parmi les dérivés anioniques comme la carboxyméthyl guar, ou encore parmi les dérivés mixtes non ioniques/anioniques comme la carboxy-hydroxypropyl guar ou les dérivés mixtes non ioniques/cationiques, et/ou leurs mélanges.

De préférence, le dérivé de guar est choisi parmi les dérivés non ioniques de la guar et de préférence encore, le dérivé de guar est l'hydroxypropyl guar.

Le dérivé de galactomannane est généralement présent dans les compositions de l'invention à une teneur allant de 0,01 à 5 %, en poids, par rapport au poids total de la composition, de préférence de 0,1 à 1% en poids, par rapport au poids total de la composition.

La phase aqueuse des compositions selon l'invention peut comprendre, outre l'agent hydratant et le dérivé de galactomannane ci-dessus, de l'eau, une eau florale telle que l'eau de bleuet et/ou une eau minérale telle que l'eau de VITTEL, l'eau de LUCAS ou l'eau de LA ROCHE POSAY et/ou une eau thermale et/ou tout composé hydrosoluble habituellement utilisé dans les compositions cosmétiques.

La composition de l'invention peut ainsi comprendre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La phase aqueuse est de préférence présente dans la composition à une teneur supérieure ou égale à 30% en poids, par rapport au poids total de la composition.

Les compositions de invention contiennent en outre un milieu cosmétiquement, physiologiquement ou dermatologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.

Les compositions de invention peuvent ainsi se présenter sous la forme d'une émulsion huile-dans-eau (H/E), une émulsion eau-dans-huile (E/H), une émulsion multiple ou une solution multiphasée. Elles peuvent également se présenter sous la forme d'un gel aqueux, d'une solution aqueuse, hydroalcoolique ou multiphasée.

Les compositions de l'invention peuvent également se présenter sous la forme d'une dispersion vésiculaire, par exemple sous la forme d'une phase huileuse dispersée dans une phase aqueuse et stabilisée par des liposomes.

Les compositions selon l'invention peuvent ainsi comprendre une phase grasse qui peut notamment être constituée de corps gras liquides à 25 °C, tels que des huiles d'origine animale, végétale, minérale ou synthétique.

La phase grasse peut comprendre toute huile cosmétiquement acceptable, dans la mesure où ladite huile permet, en mélange avec la phase aqueuse et les éventuels additifs, l'obtention d'une émulsion stable, c'est-à-dire d'une émulsion qui ne casse pas, qui reste sous forme d'une phase unique pendant au moins 24 heures après stockage à 25 °C, sans phénomène de crémage ou de relarguage d'huile.

Les huiles susceptibles d'être employées peuvent éventuellement être volatiles à température ambiante (20-25 °C). On entend par huile volatile, tout composé susceptible de s'évaporer au contact de la peau. De préférence, on utilise des huiles dont le point éclair est suffisamment élevé pour permettre l'utilisation de ces huiles en formulation.

Ces composés volatils peuvent être choisis en particulier parmi les huiles hydrocarbonées et/ou les huiles siliconées, cycliques ou linéaires, seules ou en mélange. On peut ainsi citer les huiles siliconées volatiles, telles que :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE, qui est un cyclo-copolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.

On peut également citer les huiles volatiles hydrocarbonées, telles que les isoparaffines et notamment l'isododécane.

Parmi les huiles non volatiles, on peut citer:
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, telles que l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras et de polyol, en particulier les triglycérides liquides; des alcools; des acétylglycérides; des octanoates, décanoates ou ricinoléates d'alcools ou de polyalcools; des triglycérides d'acides gras; des glycérides, les huiles fluorées et perfluorées;
- leurs mélanges.

La composition selon invention peut comprendre en outre d'autres corps gras, qui peuvent être choisis par l'homme du métier sur la base de ses connaissances générales, de manière à conférer à la composition finale les propriétés souhaitées, par exemple en consistance, en texture et/ou en transfert. Ces corps gras additionnels peuvent être des cires, des gommes et/ou des corps gras pâteux d'origine animale, végétale, minérale ou synthétique, ainsi que leurs mélanges.

On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées; leurs mélanges.

Lorsque la composition selon l'invention se présente sous la forme d'une émulsion, elle peut éventuellement comprendre en outre un tensioactif. Comme tensioactif H/E, on peut citer notamment (CTFA): le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate, le stéarate de triéthanolamine, le stéarate de sodium.

Comme tensioactif E/H, on peut citer notamment le mélange polyglycéryl-4 isostéarate/ cétyldiméthicone copolyol/ hexyl laurate, le mélange Mineral oil/petrolatum/ozokérite/glycéryl oléate/alcool de lanoline, les diméthicone copolyols, les cétyl diméthicone copolyols, les diméthicone copolyols substitués en α-ω, c'est-à-dire aux deux extrémités de la chaîne siliconée.

Dans une forme préférée de réalisation, la composition selon l'invention se présente sous la forme d'une émulsion eau-dans-huile (E/H).

La composition peut comprendre en outre tout composé complémentaire usuellement utilisé dans le domaine cosmétique, tel que des antioxydants, des parfums, des huiles essentielles, des conservateurs, des actifs cosmétiques ou pharmaceutiques lipophiles ou hydrophiles, des vitamines, des acides gras essentiels, des sphingolipides, des composés auto-bronzants tels que la DHA, des filtres solaires.

Bien entendu l'homme du métier veillera à choisir ce ou ces éventuels composés complémentaires, et/ou leur quantité, de manière telle que les propriétés avantageuses de la composition selon invention ne soient pas, ou substantiellement pas, altérées par l'adjonction envisagée.

Ces composés complémentaires peuvent être présents dans la composition à raison de 0-10 % en poids. Selon leur nature, ils sont présents dans la phase aqueuse ou dans la phase grasse de la composition.

Les compositions selon l'invention peuvent se présenter sous toutes les formes appropriées pour une application topique, notamment sous forme de stick, de lotion, de crème, de lait, de gel aqueux, d'émulsions obtenues par dispersion d'une phase grasse dans une phase aqueuse (H/E) ou inversement (E/H), de consistance liquide ou semi-liquide, voire pâteuse ou solide.

De préférence, les compositions selon l'invention se présentent sous la forme solide et présentent une dureté caractérisée par la force de pénétration nécessaire pour faire pénétrer un mobile sélectionné d'une profondeur de 2 mm au sein de la composition, et ce à à une vitesse particulière.

Dans la présente demande, cette force de pénétration est mesurée selon le protocole suivant : en fin de préparation de la composition, cette dernière est coulée dans une coupelle et est maintenue 24 heures à 20° C. On mesure alors sur cette composition solide, à l'aide d'un appareil analyseur de texture de marque STEVENS, la force de pénétration nécessaire à un mobile de mesure TA24 et de diamètre de 4 mm, pour pénétrer d'une profondeur de 2 mm dans la composition, à une vitesse de pénétration de 0,5 mm/s. La force de pénétration exprimée en grammes se lit sur l'appareil.

De préférence, les compositions selon l'invention présentent une dureté caractérisée par une force de pénétration, mesurée comme ci-dessus, supérieure ou égale à 50 g (grammes).

De préférence, les composition de l'invention sont des émulsions E/H solides qui présentent une dureté caractérisée par une force de pénétration supérieure ou égale à 50 g.

Les compositions selon l'invention peuvent se présenter sous la forme d'un produit cosmétique de maquillage, notamment de la peau et/ou des muqueuses et/ou le cuir chevelu, en particulier pour le corps et/ou le visage, comme par exemple un fond de teint, un anticerne, un produit de maquillage pour le corps, un fard à joues ou à paupières, un eye-liner, un mascara ou un rouge à lèvres.

Les compositions selon l'invention peuvent être préparées selon les méthodes de préparation classiques des émulsions E/H, H/E, des gels aqueux, ces méthodes étant bien connues de l'homme du métier.

L'invention est illustrée plus en détails dans l'exemple suivant.

### EXEMPLE 1 :

La Demanderesse a préparé six émulsions E/H de composition de base suivante :

### Composition de base :

- huile de jojoba hydrogénée 5,5 g
- isoéicosane 7 g
- cyclométhicone 11,6 g
- cire de polyéthylène 0,8 g
- sulfate de magnésium 1 g
- bentone 0,5 g
- mélange cétyl diméthicone copolyol /polyglycéryl-4-isostéarate / hexyl laurate vendu sous la dénomination commerciale "Abil WE 09" par la Société Goldschmidt 9 g
- hydroxy propyl guar vendue sous la dénomination commerciale "Jaguar HP-105" par Rhône-Poulenc Chimie X g
- phase pulvérulente Y g
- glycérine Z g
- eau 33,4 g
- conservateurs qs

Les différentes quantités en poids respectivement en hydrox propyl guar (dérivé de galactomannane), glycérine (hydratant) et phase pulvérulente sont résumées dans le tableau I ci-dessous :

**Tableau I :**

| Composition | hydroxypropylguar(X en g) | glycérine (Z en g) | phase pulvérulente (Y en g) |
|---|---|---|---|
| A (comparative) | 0,5 | 0 | 0 |
| B (comparative) | 0,5 | 0 | 26 |
| **C (invention)** | **0,5** | **3** | **26** |
| D (comparative) | 0 | 0 | 0 |
| E (comparative) | 0 | 0 | 26 |
| F (comparative) | 0 | 3 | 26 |

### La composition de la phase pulvérulente est la suivante :

Phase pulvérulente : les quantités sont données en pourcentage de poids par rapport au poids total de la phase pulvérulente :
- poudre de polyéthylène 15,5%
- nylon 11,5%
- pigments (oxydes de fer et oxydes de titane) 61,5%
- polyméthylsilsesquioxane 11,5%

Les compositions ci-dessus ont été préparées classiquement en préparant d'abord la phase grasse puis en y incorporant l'éventuelle phase pulvérulente. La phase aqueuse a ensuite été ajoutée sous agitation : le tout a ensuite été coulé.

La composition C selon invention présente une dureté dont la force de pénétration, mesurée comme décrit dans la présente demande, est supérieure à 50 g.

Pour chacune de ces compositions, on a mesuré l'hydratation cutanée conférée selon la méthode suivante : avant toute application de produit, on essuie à l'aide d'un mouchoir en papier les zones de peau à mesurer, à savoir les faces internes des avant-bras secs de 14 personnes, afin d'éliminer tout excès de sébum ou de sueur. Puis, à ce temps T0, on effectue une première mesure de l'hydratation cutanée en appliquant, perpendiculairement à ces zones de peau, la sonde préalablement essuyée d'un cornéomètre CM820 SEI-M60015-CORN.05.

Chaque composition est ensuite appliquée à raison de 1 mg/cm² sur les zones de peau à mesurer. Au bout d'une heure (T1), ces zones sont de nouveau essuyées avec un mouchoir en papier. On effectue alors une deuxième mesure de l'hydratation cutanée à ce temps T1 à l'aide de la sonde préalablement essuyée du cornéomètre.

Les valeurs lues au cornéomètre correspondent à la capacité électrique de la zone de peau mesurée. Plus ces valeurs sont élevées, plus la quantité d'eau sur la peau est grande.

Les moyennes des valeurs lues au cornéomètre sont rassemblées dans les tableaux Ila et llb suivants :

**Tableau IIa**

| Composition | T0 | T1 |
|---|---|---|
| Peau nue | 71,21 ± 5,56 | 69,64 ± 4,02 |
| D | 70,31 ± 5,16 | 69,43 ± 3,50 |
| E | 69,36 ± 5,29 | 61,71 ± 3,42 |
| B | 70,21 ± 4,87 | 62,90 ± 3,47 |

Ce premier tableau montre l'effet desséchant sur la peau des particules solides généralement présentes dans les compositions de maquillage. En effet, on voit que la composition D, qui ne comprend ni particule solide, ni hydratant, ni dérivé de guar, modifie très peu l'hydratation de la peau par rapport à la peau nue. En revanche, pour la composition E, qui comprend des particules solides telles que des charges et des pigments mais qui ne comprend ni hydratant ni dérivé de guar, on peut voir que la valeur de l'hydratation cutanée passe d'environ 69 à environ 61 ce qui représente une baisse significative de l'hydratation de la peau. On voit également avec la composition B que l'ajout d'un dérivé de guar seul ne permet pas de compenser la perte d'hydratation causée par la présence des particules solides (on passe d'une valeur d'environ 70 à une valeur d'environ 62).

**Tableau IIb**

| Composition | T0 | T1 |
|---|---|---|
| F | 69,24 ± 4,63 | 65,79 ± 3,87 |
| C | 70,55 ± 5,54 | 68,98 ± 3,70 |

Ce deuxième tableau montre que si l'on rajoute un hydratant à une composition comprenant des particules solides (composition F), on limite la perte en hydratation due à ces particules solides (on passe d'une valeur d'environ 69 à une valeur d'environ 65). Toutefois, la peau perd toujours de l'eau et donc de l'hydratation. En revanche, le fait d'ajouter un dérivé de guar (composition C selon l'invention) permet de limiter significativement cette perte.

Le pourcentage de l'évolution de l'hydratation de la peau traitée par une des compositions A à F, par rapport à la peau nue, sur une durée d'une heure, est résumée dans le tableau suivant :

| Composition | T1 |
|---|---|
| A (comparative) | 0% |
| E (comparative) | - 9% |
| B (comparative) | - 8% |
| F (comparative) | - 3% |
| C (invention) | 0% |

Il ressort de ce tableau que, bien que le dérivé de guar seul n'ait aucun effet sur l'hydratation de la peau (composition A), l'association "glycérine + dérivé de guar" dans une composition comprenant des particules solides desséchantes (composition C) permet une hydratation de la peau significativement accrue par rapport à l'ajout de la glycérine seule dans la même composition (composition F).

## Revendications

1. Composition cosmétique de maquillage, comprenant au moins une phase pulvérulente et au moins une phase aqueuse, **caractérisée par le fait que** ladite phase aqueuse comprend au moins un agent hydratant présent en une teneur allant de 0,1 à 20 % en poids, par rapport au poids total de la composition, et au moins un dérivé de galactomannane, la phase pulvérulente comprenant des particules solides choisies parmi les pigments, les nacres et/ou leurs mélanges.

2. Composition selon la revendication 1, **caractérisée par le fait que** la phase pulvérulente comprend des particules solides de taille moyenne pouvant aller jusqu'à 0,5 mm, de préférence allant de 5 nanomètres à 200 microns.

3. Composition selon la revendication 1 ou 2, **caractérisée par le fait que** la phase pulvérulente comprend des particules solides choisies parmi les charges.

4. Composition selon l'une quelconque des revendications précédentes, **caractérisée par le fait que** les pigments sont choisis parmi les dioxydes de titane, de forme sphérique ou en paillettes, les dioxydes de zirconium ou de cérium, les oxydes de zinc, de fer ou de chrome, les nanotitanes, le bleu ferrique, le noir de carbone, les sels de calcium, de baryum, d'aluminium ou de zirconium, de colorants acides tels que les colorants halogéno-acides, azoïques ou anthraquinoniques, les pigments enrobés par des composés siliconés tels que des PDMS et/ou par des polymères, les pigments interférentiels et leurs mélanges.

5. Composition selon l'une quelconque des revendications précédentes, **caractérisée par le fait que** les pigments sont présents à raison de 0-30 %, et de préférence à raison de 2-15 %, en poids, par rapport au poids total de la composition.

6. Composition selon l'une quelconque des revendications 3 à 5, **caractérisée par le fait que** les charges sont choisies parmi le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques, les microéponges, les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, comme le stéarate de zinc, de magnésium ou de lithium, le laurate de zinc, le myristate de magnésium, les composés SiO₂/TiO₂/ SiO₂, TiO₂/CeO₂/SiO₂, ou encore TiO₂/ZnO/Talc, les polymères de polyéthylène terephtalate/polyméthacrylate en forme de paillettes.

7. Composition selon l'une quelconque des revendications 3 à 6, **caractérisée par le fait que** les charges sont présentes dans la composition à raison de 0-20 % en poids, de préférence 2-10 %, par rapport au poids total de la composition.

8. Composition selon l'une quelconque des revendications précédentes, **caractérisée par le fait que** les nacres sont choisies parmi la nacre naturelle, le mica recouvert d'oxyde de titane, d'oxyde de fer, de pigment naturel ou d'oxychlorure de bismuth ainsi que le mica titane coloré.

9. Composition selon l'une quelconque des revendications précédentes, **caractérisée par le fait que** les nacres sont présentes dans la composition à raison de 0-20 % en poids, de préférence de 2-15 % en poids, par rapport au poids total de la composition.

10. Composition selon l'une quelconque des revendications précédentes, **caractérisée par le fait que** la phase pulvérulente est présente à une teneur allant de 0,1 à 50%, de préférence de 5 à 30% en poids, par rapport au poids total de la composition.

11. Composition selon l'une quelconque des revendications précédentes, **caractérisée par le fait que** l'agent hydratant est choisi parmi la glycérine, le propylène glycol, l'hexylene glycol, le butylène glycol, le pentylène glycol, le dipropylène glycol, le sorbitol, le mannitol, le xylitol, les polyols, les polyéthylène glycols et/ou leurs mélanges.

12. Composition selon l'une quelconque des revendications précédentes, **caractérisée par le fait que** l'agent hydratant est la glycérine.

13. Composition selon l'une quelconque des revendications précédentes, **caractérisée par le fait que** l'agent hydratant est présent à une teneur allant de 2 à 10% en poids, par rapport au poids total de la composition.

14. Composition selon l'une quelconque des revendications précédentes, **caractérisée par le fait que** le dérivé de galactomannane est un polysaccharide d'origine végétale, hydrophile, comprenant essentiellement une répétition de motifs β-D-mannoses formant une chaîne principale sur laquelle sont branchés des motifs α-D-galactoses.

15. Composition selon l'une quelconque des revendications précédentes, **caractérisée par le fait que** le dérivé de galactomannane est choisi parmi les dérivés du guar et les dérivés de la gomme de caroube.

16. Composition selon l'une quelconque des revendications précédentes, **caractérisée par le fait que** le dérivé de galactomannane est choisi parmi les dérivés de guar.

17. Composition selon la revendication 16, **caractérisée par le fait que** le dérivé de guar est choisi parmi les dérivés cationiques de la guar, les dérivés non ioniques de la guar, les dérivés anioniques de la guar, les dérivés mixtes non ioniques/anioniques de la guar, les dérivés mixtes non ioniques/cationiques de la guar, et/ou leurs mélanges.

18. Composition selon la revendication 16 ou 17, **caractérisée par le fait que** le dérivé de guar est choisi parmi les dérivés non ioniques de la guar.

19. Composition selon l'une quelconque des revendications précédentes 16 à 18, **caractérisée par le fait que** le dérivé de guar est l'hydroxypropyl guar.

20. Composition selon l'une quelconque des revendications précédentes, **caractérisée par le fait que** le dérivé de galactomannane est présent dans les compositions de l'invention à une teneur allant de 0,01 à 5 %, en poids, par rapport au poids total de la composition, de préférence de 0,1 à 1% en poids, par rapport au poids total de la composition.

21. Composition selon l'une quelconque des revendications précédentes, **caractérisée par le fait que** la phase aqueuse est présente à une teneur supérieure ou égale à 30% en poids, par rapport au poids total de la composition.

22. Composition selon l'une quelconque des revendications précédentes, **caractérisée par le fait qu'**elle comprend en outre une phase grasse.

23. Composition selon l'une quelconque des revendications précédentes, **caractérisée par le fait que** c'est une émulsion eau-dans-huile.

24. Composition selon l'une quelconque des revendications précédentes, **caractérisée par le fait qu'**elle est solide.

25. Composition selon la revendication précédente, **caractérisée par le fait qu'**elle présente une dureté définie par une force de pénétration supérieure ou égale à 50 g.

26. Composition selon l'une quelconque des revendications précédentes, **caractérisée par le fait que** c'est une composition de fond de teint, d'anticeme, de produit de maquillage pour le corps, de fard à joues ou à paupières, d'eye-liner, de mascara ou de rouge à lèvres.

27. Procédé non thérapeutique de maquillage de la peau et/ou des muqueuses consistant à appliquer sur la peau et/ou les muqueuses une composition telle que définie à l'une quelconque des revendications 1 à 26.

28. Utilisation d'un dérivé de galactomannane dans une composition cosmétique de maquillage, comprenant au moins une phase pulvérulente comprenant des particules solides choisies parmi les pigments, les nacres et/ou leurs mélanges, et au moins une phase aqueuse comprenant un agent hydratant présent en une teneur allant de 0,1 à 20 % en poids, par rapport au poids total de la composition, afin de diminuer et/ou limiter l'effet desséchant et/ou améliorer le pouvoir hydratant de ladite composition.

## Patentansprüche

1. Kosmetische Schminkzusammensetzung, die mindestens eine pulverförmige Phase und mindestens eine wässrige Phase enthält, **dadurch gekennzeichnet, dass** die wässrige Phase mindestens ein Hydxatisierungsmittel, das in einem Anteil, der im Bereich von 0,1 bis 20 Gew.-% liegt, bezogen auf das Gesamtgewicht der Zusammensetzung, und mindestens ein Galactomannan-Derivat enthält, wobei die pulverförmige Phase feste Partikel enthält, die unter den Pigmenten, den Perlglanzpigmenten und/oder deren Gemischen ausgewählt sind.

2. Zusammensetzung nach Anspruch 1, **dadurch gekennzeichnet, dass** die pulverförmige Phase feste Partikel enthält, deren mittlere Größe bis zu 0,5 mm betragen kann und vorzugsweise im Bereich von 5 nm bis 200 µm liegt.

3. Zusammensetzung nach Anspruch 1 oder 2, **dadurch gekennzeichnet, dass** die pulverförmige Phase feste Partikel enthält, die unter den Füllstoffen ausgewählt sind.

4. Zusammensetzung nach einem der vorhergehenden Ansprüche, **dadurch gekennzeichnet, dass** die Pigmente unter den Titandioxiden in Form von Kügelchen oder Plättchen, Zirconiumdioxid, Cerdioxid, Zinkoxid, Eisenoxiden, Chromoxid, den Nanotitanen, den Eisenblau-Pigmenten, Ruß, den Calciumsalzen, Bariumsalzen, Aluminiumsalzen und Zirconiumsalzen saurer Farbstoffe, wie halogenhaltiger saurer Farbstoffe, Azofarbstoffen oder Anthrachinonfarbstoffen, den Pigmenten, die mit Siliconverbindungen, wie PDMS, und/oder Polymeren umhüllt sind, den Interferenzpigmenten und den Gemischen dieser Pigmente ausgewählt sind

5. Zusammensetzung nach einem der vorhergehenden Ansprüche, **dadurch gekennzeichnet, dass** die Pigmente in einem Anteil von 0 bis 30 Gew.-% und vorzugsweise in einem Anteil von 2 bis 15 Gew.-%, bezogen auf das Gesamtgewicht der Zusammensetzung, enthalten sind.

6. Zusammensetzung nach einem der Ansprüche 3 bis 5, **dadurch gekennzeichnet, dass** die Füllstoffe ausgewählt sind unter Talk, Glimmer, Kieselsäure, Kaolin, Nylon-, Poly-β- und Polyethylen-Pulvern, Teflon, Lauroyllysin, Stärke, Bornitrid, Bismutoxidchlorid, Tetrafluorethylenpolymer-Pulvern, Polymethylmethacrylat-Pulvern, Polyurethanpulvern, Polystyrolpulvern, Polyesterpulvern, synthetischen hohlen Mikrokügelchen, Mikroschwämmen, Mikrokügelchen aus Silcionharz, Zinkoxid, Titandioxid, Zirconiumdioxid, Cerdioxid, gefälltem Calciumcarbonat, Magnesiumcarbonat, Magnesiumhydrogencarbonat, Hydroxyapatit, hohlen Mikrokügelchen aus Kieselsäure, Mikrokapseln aus Glas oder Keramik, Metallseifen, die von organischen Carbonsäuren abgeleitet sind, die 8 bis 22 Kohlenstoffatome, vorzugsweise 12 bis 18 Kohlenstoffatome, aufweisen, wie Zinkstearat, Magnesiumstearat oder Lithiumstearat, Zinklaurat, Magnesiummyristat, den Verbindungen SiO₂/TiO₂/SiO₂, TiO₂/CeO₂/SiO₂ oder TiO₂/ZnO₂/Talk, den Polyethylenterepthalat/Polymethacrylat, Polymeren in Form von Plättchen.

7. Zusammensetzung nach einem der Ansprüche 3 bis 6, **dadurch gekennzeichnet, dass** die Füllstoffe in der Zusammensetzung in einem Anteil von 0 bis 20 Gew,-%, vorzugsweise 2 bis 10 Gew.-%, bezogen auf das Gesamtgewicht der Zusammensetzung, enthalten sind.

8. , Zusammensetzung nach einem der vorhergehenden Ansprüche, **dadurch gekennzeichnet, dass** die Perlglanzpigmente unter Perlmutt, Glimmer, der mit Titandioxid, Eisenoxid, natürlichem Pigment oder Bismutoxidchlorid überzogen ist, sowie farbigem Titandioxidglimmer ausgewählt sind.

9. Zusammensetzung nach einem der vorhergehenden Ansprüche, **dadurch kennzeichnet, dass** die Perlglanzpigmente in der Zusammensetzung in einem Anteil von 0 bis 20 Gew.-%, vorzugsweise 2 bis 15 Gew,-%, bezogen auf das Gesamtgewicht der Zusammensetzung, enthalten sind.

10. Zusammensetzung nach einem der vorhergehenden Ansprüche, **dadurch kennzeichnet, dass** die pulverförmige Phase in einem Anteil von 0,1 bis 50 Gew.-%, vorzugsweise 5 bis 30 Gew.-%, bezogen auf das Gesamtgewicht der Zusammensetzung, enthalten ist.

11. Zusammensetzung nach einem der vorhergehenden Ansprüche, **dadurch kennzeichnet, dass** das Hydratisierungsmittel unter Glycerin, Propylenglykol, Hexylenglykol, Butylenglykol, Pentylenglykol, Dipropylenglykol, Sorbit, Mannit, Xylit, den Polyolen, den Polyethylenglykolen und/oder ihren Gemischen ausgewählt ist.

12. Zusammensetzung nach einem der vorhergehenden Ansprüche, **dadurch kennzeichnet, dass** es sich bei dem Hydratisierungsmittel um Glycerin handelt.

13. Zusammensetzung nach einem der vorhergehenden Ansprüche, **dadurch kennzeichnet, dass** das Hydratisierungsmittel in einem Anteil enthalten ist, der im Bereich von 2 bis 10 Gew.-%, bezogen auf das Gesamtgewicht der Zusammensetzung, liegt.

14. Zusammensetzung nach einem der vorhergehenden Ansprüche, **dadurch kennzeichnet, dass** das Galactomannan-Derivat ein hydrophiles Polysaccharid pflanzlicher Herkunft ist, das im wesentlichen eine Aneinanderreihung von β-D-Mannose-Einheiten umfasst, die eine Hauptkette bilden, an die α-D-Galactose-Einheiten als Seitengruppen angebunden sind.

15. Zusammensetzung nach einem der vorhergehenden Ansprüche, **dadurch kennzeichnet, dass** das Galactomannan-Derivat unter den Guar-Derivaten und den Carobengummi-Derivaten ausgewählt ist.

16. Zusammensetzung nach einem der vorhergehenden Ansprüche, **dadurch kennzeichnet, dass** das Galactomannan-Derivat unter den Guar-Derivaten ausgewählt ist.

17. Zusammensetzung nach Anspruch 16, **dadurch gekennzeichnet, dass** das Galactomannan-berivat unter den kationischen Guar-Derivaten, den nichtionischen Guar-Derivaten, den anionischen Guar-Derivaten, den gemischten nichtionischen/anionischen Guar-Derivaten, den gemischten nichtionischen/kationischen Guar-Derivaten und/oder ihren Gemischen ausgewählt ist.

18. Zusammensetzung nach Anspruch 16 oder 17, **dadurch gekennzeichnet, dass** das Guar-Derivat unter den nichtionischen Guar-Derivaten ausgewählt ist.

19. Zusammensetzung nach einem der vorhergehenden Ansprüche 16 bis 18, **dadurch gekennzeichnet, dass** es sich bei dem Guar-Derivat um Hydroxypropylguar handelt.

20. Zusammensetzung nach einem der vorhergehenden Ansprüche, **dadurch gekennzeichnet, dass** das Galactomannan-Derivat in den erfindungsgemäßen Zusammensetzungen in einem Anteil von 0,01 bis 5 Gew.-%, bezogen auf das Gesamtgewicht der Zusammensetzung, und vorzugsweise in einem Anteil von 0,1 bis 1 Gew.-%, bezogen auf das Gesamtgewicht der Zusammensetzung, enthalten ist.

21. Zusammensetzung nach einem der vorhergehenden Ansprüche, **dadurch gekennzeichnet, dass** die wässrige Phase in einem Anteil von 30 Gew.-% oder darüber, bezogen auf das Gesamtgewicht der Zusammensetzung, enthalten ist.

22. Zusammensetzung nach einem der vorhergehenden Ansprüche, **dadurch gekennzeichnet, dass** sie außerdem eine Fettphase enthält.

23. Zusammensetzung nach einem der vorhergehenden Ansprüche, **dadurch gekennzeichnet, dass** es sich um eine Wasser-in-Öl-Emulsion handelt.

24. Zusammensetzung nach einem der vorhergehenden Ansprüche, **dadurch gekennzeichnet, dass** sie fest ist.

25. Zusammensetzung nach dem vorhergehenden Anspruch, **dadurch gekennzeichnet, dass** sie eine Härte aufweist, die durch eine Eindringkraft von 50 g oder darüber definiert ist.

26. Zusammensetzung nach einem der vorhergehenden Ansprüche, **dadurch gekennzeichnet, dass** es sich um eine Make-up-Zusammensetzung, ein Produkt gegen Augenringe, ein Schminkprodukt für den Körper, ein Rouge oder Lidschatten, einen Eyeliner, Mascara oder Lippenstift handelt.

27. Nicht-therapeutisches Verfahren zum Schminken der Haut und/ oder der Schleimhäute, das darin besteht, auf die Haut und/ oder die Schleimhäute eine wie in einem der Ansprüche 1 bis 26 definierte Zusammensetzung aufzutragen.

28. Verwendung eines Galactomannan-Derivats in einer kosmetischen Schminkzusammensetzung, die mindestens eine pulverförmige Phase, die feste Partikel enthält, die unter Pigmenten, Perlglanzpigmenten und/oder deren Gemischen ausgewählt sind, und mindestens eine wässrige Phase enthält, die ein Hydratisierungsmittel enthält, das in einem Anteil enthalten ist, der im Bereich von 0,1 bis 20 Gew.-%, bezogen auf das Gesamtgewicht der Zusammensetzung, liegt, um die austrocknende Wirkung der Zusammensetzung zu verringern und/oder einzuschränken und/oder das Hydratisierungsvermögen der Zusammensetzung zu verbessern.

## Claims

1. Cosmetic make-up composition, comprising at least one pulverulent phase and at least one aqueous phase, **characterized in that** the said aqueous phase comprises at least one moisturizer present in a content ranging from 0.1% to 20% by weight, relative to the total weight of the composition, and at least one galactomannan derivative, the pulverulent phase comprising solid particles chosen from pigments, nacres, and/or mixtures thereof.

2. Composition according to Claim 1, **characterized in that** the pulverulent phase comprises solid particles with an average size which may be up to 0.5 mm and preferably ranging from 5 nanometres to 200 microns.

3. Composition according to Claim 1 or 2, **characterized in that** the pulverulent phase comprises solid particles chosen from fillers.

4. Composition according to any one of the preceding claims, **characterized in that** the pigments are chosen from titanium dioxides, of spherical shape or in the form of flakes, zirconium dioxides or cerium dioxides, zinc oxides, iron oxides, chromium oxides, nanotitaniums, ferric blue, carbon black, calcium salts, barium salts, aluminium salts or zirconium salts, acidic dyes such as halo-acid dyes, azo dyes or anthraquinone dyes, pigments coated with silicone compounds such as PDMSs and/or with polymers, and interference pigments, and mixtures thereof.

5. Composition according to any one of the preceding claims, **characterized in that** the pigments are present in a proportion of 0-30% and preferably in a proportion of 2-15% by weight relative to the total weight of the composition.

6. Composition according to any one of Claims 3 to 5, **characterized in that** the fillers are chosen from talc, mica, silica, kaolin, Nylon powder, poly-β-alanine powder, polyethylene powder, Teflon, lauroyllysine, starch, boron nitride, bismuth oxychloride, tetrafluoroethylene polymer powders, polymethyl methacrylate powders, polyurethane powders, polystyrene powders, polyester powders, synthetic hollow microspheres, microspanges, silicone resin microbeads, zinc oxides, titanium oxides, zirconium oxides or cerium oxides, precipitated calcium carbonate, magnesium carbonate, magnesium hydrocarbonate, hydroxyapatite, hollow silica microspheres, glass or ceramic microcapsules, metal soaps derived from organocarboxylic acids containing from 8 to 22 carbon atoms and preferably from 12 to 18 carbon atoms, for instance zinc stearate, magnesium stearate, lithium stearate, zinc laurate or magnesium myristate, the compounds SiO₂/TiO₂/SiO₂, TiO₂/CeO₂/SiO₂ or TiO₂/ZnO/talc, polyethylene terephthalate/polymethacrylate polymers in the form of flakes.

7. Composition according to any one of Claims 3 to 6, **characterized in that** the fillers are present in the composition in a proportion of 0-20% by weight and preferably 2-10% relative to the total weight of the composition.

8. Composition according to any one of the preceding claims, **characterized in that** the nacres are chosen from natural mother-of-pearl, mica coated with titanium oxide, with iron oxide, with natural pigment or with bismuth oxychloride, and coloured titanium mica.

9. Composition according to any one of the preceding claims, **characterized in that** the nacres are present in the composition in a proportion of 0-20% by weight and preferably 2-15% by weight relative to the total weight of the composition.

10. Composition according to any one of the preceding claims, **characterized in that** the pulverulent phase is present in a content ranging from 0.1% to 50% and preferably from 5% to 30% by weight relative to the total weight of the composition.

11. Composition according to any one of the preceding claims, **characterized in that** the moisturizer is chosen from glycerol, propylene glycol, hexylene glycol, butylene glycol, pentylene glycol, dipropylene glycol, sorbitol, mannitol, xylitol, polyols and polyethylene glycols, and/or mixtures thereof.

12. Composition according to any one of the preceding claims, **characterized in that** the moisturizer is glycerol.

13. Composition according to any one of the preceding claims, **characterized in that** the moisturizer is present in a content ranging from 2% to 10% by weight relative to the total weight of the composition.

14. Composition according to any one of the preceding claims, **characterized in that** the galactomannan derivative is a hydrophilic polysaccharide of plant origin, essentially comprising a repetition of β-D-mannose units forming a main chain onto which are branched α-D-galactose units.

15. Composition according to any one of the preceding claims, **characterized in that** the galactomannan derivative is chosen from guar derivatives and carob gum derivatives.

16. Composition according to any one of the preceding claims, **characterized in that** the galactomannan derivative is chosen from guar derivatives.

17. Composition according to Claim 16, **characterized in that** the guar derivative is chosen from cationic guar derivatives, nonionic guar derivatives, anionic guar derivatives, nonionic/anionic mixed guar derivatives and nonionic/cationic mixed guar derivatives, and/or mixtures thereof.

18. Composition according to Claim 16 or 17, **characterized in that** the guar derivative is chosen from nonionic guar derivatives.

19. Composition according to any one of the preceding Claims 16 to 18, **characterized in that** the guar derivative is hydroxypropyl guar.

20. Composition according to any one of the preceding claims, **characterized in that** the galactomannan derivative is present in the compositions of the invention in a content ranging from 0.01% to 5% by weight relative to the total weight of the composition and preferably from 0.1% to 1% by weight relative to the total weight of the composition.

21. Composition according to any one of the preceding claims, **characterized in that** the aqueous phase is present in a content of greater than or equal to 30% by weight relative to the total weight of the composition.

22. Composition according to any one of the preceding claims, **characterized in that** it also comprises a fatty phase.

23. Composition according to any one of the preceding claims, **characterized in that** it is a water-in-oil emulsion.

24. Composition according to any one of the preceding claims, **characterized in that** it is solid.

25. Composition according to the preceding claim, **characterized in that** it has a hardness, defined by a penetration force, of greater than or equal to 50 g.

26. Composition according to any one of the preceding claims, **characterized in that** it is a foundation or concealer composition, a make-up product for the body or a face powder, eyeshadow, eyeliner, mascara or lipstick composition.

27. Nontherapeutic process for making up the skin and/or mucous membranes which consists in applying to the skin and/or mucous membranes a composition as defined in any one of Claims 1 to 26.

28. Use of a galactomannan derivative in a cosmetic make-up composition, comprising at least one pulverulent phase comprising solid particles chosen form pigments, nacres, and/or mixtures thereof, and at least one aqueous phase comprising a moisturizer present in a content ranging from 0.1% to 20% by weight, relative to the total weight of the composition, in order to reduce and/or limit the drying effect and/or to improve the moisturizing power of the said composition.
